# EUROPÄISCHE PATENTSCHRIFT

(11) **EP 1 935 876 B1**
(45) Veröffentlichungstag und Bekanntmachung des Hinweises auf die Patenterteilung: **16.10.2013**
(21) Anmeldenummer: 07023224.4
(22) Anmeldetag: 30.11.2007
(51) Int. Cl.: C07C 263/10

(54) **Verfahren zur Herstellung von Isocyanaten in der Gasphase**
Method for manufacturing isocyanates in the gaseous phase
Procédé destiné à la fabrication d'isocyanates en phase gazeuse

(30) Priorität: 13.12.2006 DE 102006058633
(43) Veröffentlichungstag der Anmeldung: 25.06.2008
(73) Patentinhaber: Bayer Intellectual Property GmbH, 40789 Monheim (DE)
(72) Erfinder: Pohl, Fritz, Dr., 25541 Brunsbüttel (DE); Biskup, Klaus, Dr., 51373 Leverkusen (DE); Bruns, Rainer, Dr., 51373 Leverkusen (DE); Steffens, Friedhelm, 51373 Leverkusen (DE); Stutz, Herbert, Dr., 41541 Dormagen (DE)
(74) Vertreter: BIP Patents

(56) Entgegenhaltungen:
- WO-A-2007/014936
- WO-A-2007/028715
- US-A- 5 391 683

## Beschreibung

Die Erfindung betrifft ein Verfahren zur Herstellung von Isocyanaten durch Umsetzung entsprechender primärer Amine mit Phosgen, worin die primären Amine verdampft und nach der Verdampfung die damofförmigen Amine über einen Nacherhitzer auf die Einsatztemperatur gebracht werden, dadurch gekennzeichnet, dass Phosgen und die primären Amine oberhalb der Siedetemperatur der Amine innerhalb einer mittleren Kontaktzeit von 0,05 bis 15 Sekunden umgesetzt werden, wobei die Umsetzung adiabat durchgeführt wird.

Es sind aus dem Stand der Technik verschiedene Verfahren zur Herstellung von Isocyanaten durch Umsetzung von Aminen mit Phosgen in der Gasphase bekannt. EP-A-593 334 beschreibt ein Verfahren zur Herstellung von aromatischen Diisocyanaten in der Gasphase, dadurch gekennzeichnet, dass die Umsetzung des Diamins mit Phosgen in einem Rohrreaktor ohne bewegte Teile und mit einer Verengung der Wände entlang der Längsachse des Rohrreaktors stattfindet. Das Verfahren ist jedoch problematisch, da die Vermischung der Eduktströme allein über eine Verengung der Rohrwand schlecht im Vergleich zur Anwendung eines richtigen Mischorgans funktioniert. Eine schlechte Vermischung führt üblicherweise zu einer unerwünscht hohen Feststoffbildung. Diese Schrift offenbart nicht, die Reaktion adiabat zu führen.

EP-A-699 657 beschreibt ein Verfahren zur Herstellung von aromatischen Diisocyanaten in der Gasphase, dadurch gekennzeichnet, dass die Umsetzung des zugehörigen Diamins mit dem Phosgen in einem zweizonigen Reaktor stattfindet, wobei die erste Zone, die etwa 20 % bis 80% des Gesamtreaktorvolumens ausmacht, ideal vermischt ist und die zweite Zone, die 80% bis 20% des Gesamtreaktorvolumens ausmacht, durch eine Kolbenströmung charakterisiert werden kann. Weil jedoch mindestens 20% des Reaktionsvolumens ideal rückvermischt sind, resultiert eine ungleichmäßige Verweilzeitverteilung, die zu einer unerwünscht erhöhten Feststoffbildung führen kann. EP-A-289 840 beschreibt die Herstellung von Diisocyanaten durch Gasphasenphosgenierung, wobei die Herstellung erfindungsgemäß in einer turbulenten Strömung bei Temperaturen zwischen 200°C und 600°C in einem zylindrischen Raum ohne bewegte Teile stattfindet. Durch den Verzicht auf bewegte Teile wird die Gefahr eines Phosgenaustrittes reduziert. Durch die turbulente Strömung im zylindrischen Raum ( Rohr ) wird, wenn man von Fluidelementen in Wandnähe absieht, eine relative gute Strömungsgleichverteilung im Rohr und damit eine relativ enge Verweilzeitverteilung erreicht, die, wie in EP-A-570 799 beschrieben, zu einer Verringerung der Feststoffbildung führen kann.

EP-A- 570 799 betrifft ein Verfahren zur Herstellung von aromatischen Diisocyanaten, dadurch gekennzeichnet, dass die Umsetzung des zugehörigen Diamins mit dem Phosgen in einem Rohrreaktor oberhalb der Siedetemperatur des Diamins innerhalb einer mittleren Kontaktzeit von 0,5 bis 5 Sekunden durchgeführt wird. Wie in der Schrift beschrieben ist, führen zu lange als auch zu kurze Reaktionszeiten zu einer unerwünschten Feststoffbildung. Es wird daher ein Verfahren offenbart, bei dem die mittlere Abweichung von der mittleren Kontaktzeit weniger als 6% beträgt. Die Einhaltung dieser Kontaktzeit wird dadurch erreicht, dass die Reaktion in einer Rohrströmung durchgeführt wird, die entweder durch eine Reynolds-Zahl von oberhalb 4.000 oder eine Bodenstein-Zahl von oberhalb 100 charakterisiert wird.

EP-A-749 958 beschreibt ein Verfahren zur Herstellung von Triisocyanaten durch Gasphasenphosgenierung von (cyclo)aliphatischen Triaminen mit drei primären Amingruppen, dadurch gekennzeichnet, dass man das Triamin und das Phosgen kontinuierlich in einem auf 200° bis 600°C erhitzten, zylindrischen Reaktionsraum mit einer Strömungsgeschwindigkeit von mindestens 3 m/s miteinander zur Reaktion bringt.

EP-A-928 785 beschreibt die Verwendung von Mikrostrukturmischern zur Phosgenierung von Aminen in der Gasphase. Nachteilig bei der Verwendung von Mikromischern ist, dass bereits kleinste Feststoffmengen, deren Entstehung bei der Synthese der Isocyanate nicht vollständig auszuschließen ist, zum Verstopfen des Mischers führen können, was die zeitliche Verfügbarkeit der Phosgenierungsanlage herabsetzt.

Ausführlich behandelt WO 03/045900 die Herstellung von Isocyanaten im großtechnischen Maßstab unter Anwendung von Gasphasenphosgenierung. Wie in WO 03/ 045900 dargestellt, bieten sich bei den bekannten Verfahren zur Gasphasenphosgenierung, die einen zylindrischen Reaktionsraum verwenden, zwei Möglichkeiten der technischen Realisierung an. Zum einen kann die Reaktion in einer einzigen Rohrstrecke durchgeführt werden, deren Durchmesser an die Produktionskapazität der Anlage angepasst werden muss. Gemäß WO03/045900 hat dieses Konzept für sehr gro-ße Produktionsanlagen den Nachteil, dass eine genaue Temperierung der Reaktionsströme im Kern der Strömung durch eine Wandheizung des Rohres nicht mehr realisierbar ist, lokale Temperatur-Inhomogenitäten aber bei zu hoher Temperatur zur Produktzersetzung, bei zu niedriger Temperatur zur ungenügenden Umsetzung der Edukte zum gewünschten Isocyanat führen können.

Auch die zweite Möglichkeit der technischen Realisierung, die Aufteilung des reagierenden Gemisches in einzelne Teilströme, die dann parallel durch kleinere, einzelne Rohre geleitet werden, die auf Grund ihres kleineren Durchmessers besser temperierbar sind, wird von WO03/045900 als nachteilig angesehen. Gemäß WO03/045900 ist bei dieser Verfahrensvariante nachteilig, dass diese Variante relativ verstopfungsanfällig ist, wenn nicht der Volumenstrom durch jedes einzelne Rohr geregelt wird. Zur Begründung führt WO03/045900 aus, dass, wenn eines der Rohre an einer Stelle eine Ablagerung bekommt, der Druckverlust der Strömung durch dieses Rohr größer wird, das Reaktionsgas dann automatisch verstärkt auf andere Rohre ausweicht. Dies mit der Folge, dass das Rohr mit den Ablagerungen weniger durchströmt wird, mithin die Strömung durch das Rohr eine verlängerte Verweilzeit erfährt, was, wie bereits in EP-A-570 799 erklärt, zu einer Erhöhung der Feststoffbildung führt.

Zusammenfassend führt WO03/045900 aus, dass bei großtechnischen Gasphasenphosgenierungen die Verwendung eines großen Rohres das Problem der Temperierung der Gesamtströmung und die Verwendung vieler kleiner Rohre die Gefahr der ungleichmäßigen Durchströmung aufweist.

Nach der Lehre von WO03/045900 können die skizzierten Nachteile dadurch umgangen werden, dass die kontinuierliche Phosgenierung von Aminen in der Gasphase vorteilhaft, d.h. beispielsweise bei wesentlich erhöhter Betriebsstundenzahl der Produktionsanlage, durchgeführt werden kann, wenn die Reaktion in einem nicht zylindrischen Reaktionskanal, bevorzugt einem Plattenreaktor, durchgeführt wird, der bevorzugt eine Höhe aufweist, welche eine vorteilhafte Temperierung der Reaktanden ermöglicht und der eine Breite aufweist, die mindestens das zweifache der Höhe beträgt. Wie WO03/045900 weiter ausführt, ist die Höhe des Reaktionskanals im allgemeinen nicht beschränkt, kann die Umsetzung in einem Reaktionskanal mit einer Höhe von beispielsweise 40 cm durchgeführt werden, soll jedoch ein besserer Wärmeaustausch mit den Reaktorwänden erfolgen, lehrt WO03/045900 die Umsetzung in Reaktionskanälen mit geringer Höhe, beispielsweise nur einige Zentimeter oder Millimeter, mithin von Reaktordimensionen, bei denen - wie WO03/045900 bei der Kommentierung von EP-928 758 hinweist - bereits kleinste Feststoffmengen, deren Entstehung bei der Synthese der Isocyanate nicht vollständig auszuschließen ist, zum Verstopfen des Reaktors führen können, was die zeitliche Verfügbarkeit der Phosgenierungsanlagen herabsetzt.

WO 2007/014936 offenbart ein Verfahren zur Herstellung von Diisocyanaten durch Umsetzung der korrespondierenden Diamine mit Phosgen im stöchiometrischen Überschuss von Phosgen in mindestens einer Reaktionszone, wobei man die Reaktionsbedingungen so wählt, dass zumindest die Reaktionskomponenten Diamin, Diisocyanat und Phosgen bei diesen Bedingungen gasförmig sind, und wobei man der Reaktionszone mindestens einen diaminhaltigen und mindestens einen phosgenhaltigen Gasstrom zuführt, wobei man aus dem erhaltenen, im wesentlichen gasförmigen Reaktionsgemisch überschüssiges Phosgen und entstandenes Chlorwasserstoffgas (HCI) abtrennt, man das abgetrennte, überschüssige Phosgen zumindest teilweise wieder in die Reaktion zurückführt, und wobei man aus dem zurückgeführten Phosgenstrom Chlorwasserstoff so abtrennt, dass der Massenbruch von Chlorwasserstoff im phosgenhaltigen Strom vor der Vermischung mit dem aminhaltigen Strom weniger als 15 Gew.-% beträgt. Die Umsetzung von Diamin mit Phosgen kann dabei adiabat erfolgen. Diese Schrift offenbart nicht, den durch Verdampfung des Diamins erhaltenen dampfförmigen Diaminstrom über einen Nacherhitzer auf die Einsatztemperatur zu bringen.

WO2007/028715 offenbart ein Verfahren zur Herstellung von Isocyanaten durch Umsetzung der korrespondierenden Amine mit Phosgen, gegebenenfalls in Gegenwart von mindestens einem Inertmedium, in der Gasphase, in dem in einem Reaktor *n* Aminströme mit *n*+*1* Phosgenströmen umgesetzt werden, wobei *n* eine positive ganze Zahl von mindestens 1 ist, und alle Amin- und Phosgenströme jeweils über Ringspalte zur Vermischung in den Reaktor dosiert werden. Die Umsetzung von Amin mit Phosgen kann dabei adiabat erfolgen. Diese Schrift offenbart nicht, den durch Verdampfung des Amins erhaltenen dampfförmigen Aminstrom über einen Nacherhitzer auf die Einsatztemperatur zu bringen.

Überraschenderweise wurde nun gefunden, dass die Herstellung von Isocyanaten durch die Umsetzung entsprechender primärer Amine mit Phosgen in der Gasphase bei Beachtung einer mittleren Verweilzeit im Reaktionsraum von 0,05 bis 15 s unter adiabater Reaktionsführung durchgeführt werden kann. So können vorteilhaft und unabhängig von der Reaktorgeometrie Temperierungsprobleme vermieden werden und die Isocyanate im großtechnischen Maßstab mit hohen Raum/Zeit-Ausbeuten und wesentlich erhöhter Betriebsstundenzahl der Produktionsanlagen gewonnen werden.

Gegenstand der Erfindung ist daher ein Verfahren zur Herstellung von Isocyanaten durch Umsetzung entsprechender primärer Amine mit Phosgen, worin die primären Amine verdampft und nach der Verdampfung die dampfförmigen Amine über einen Nacherhitzer auf die Einsatztemperatur gebracht werden, dadurch gekennzeichnet, dass Phosgen und die primären Amine oberhalb der Siedetemperatur der Amine, d.h. in der Gasphase, innerhalb einer mittleren Kontaktzeit von 0,05 bis 15 Sekunden umgesetzt werden, wobei die Umsetzung adiabat durchgeführt wird.

Bevorzugt weist das erfindungsgemäße Verfahren einen oder mehrere der folgenden Schritte a) - d) auf, wobei besonders bevorzugt alle Schritte a) - d) durchgeführt werden: Die Schritte a) - d) sind dadurch gekennzeichnet, dass man
a) die dampfförmigen Amine, gegebenenfalls verdünnt mit einem Inertgas oder mit den Dämpfen eines inerten Lösungsmittels, und Phosgen getrennt auf Temperaturen von 200 bis 600 °C erhitzt und kontinuierlich vermischt,
b) das erzeugte Reaktionsgemisch unter Vermeidung von Rückvermischung kontinuierlich durch einen Reaktionsraum leitet und es darin innerhalb einer mittleren Kontaktzeit von 0,05 bis 15 Sekunden adiabat umsetzt,
c) das aus dem Reaktionsraum austretende Gasgemisch zur Kondensation des gebildeten Isocyanats abkühlt, wobei die Temperatur oberhalb der Zersetzungstemperatur der den umgesetzten Aminen entsprechenden Carbamidsäurechloride gehalten wird,
d) nicht kondensiertes Isocyanat aus dem Gasgemisch durch Waschen mit einer Waschflüssigkeit abtrennt.

Bevorzugt wird in Schritt b) ein Reaktionsraum eingesetzt, der eine rotationssymmetrische Geometrie mit einer in Strömungsrichtung des Reaktionsgemischs konstanten oder steigenden durchströmten Querschnittsfläche aufweist. Bevorzugt wird als Reaktionsraum ein Rohrreaktor mit einer in Strömungsrichtung des Reaktionsgemischs im Wesentlichen konstanten oder steigenden durchströmten Querschnittsfläche eingesetzt. In einer weiteren bevorzugten Ausführungsform weist der Reaktionsraum, bevorzugt ein Rohrreaktor, in Strömungsrichtung Abschnitte konstanter und steigender Querschnittsfläche auf.

Die erfindungsgemäß geeignete Ausführung des Reaktionsraums mit rotationssymmetrischer Geometrie und einer in Strömungsrichtung kaskadenförmigen und/oder kontinuierlichen Veränderung der durchströmten Querschnittsfläche hat den Vorteil, dass die Strömungsgeschwindigkeit entlang der Achse des Reaktionsraums eingestellt werden kann. Eine in Strömungsrichtung konstante durchströmte Querschnittsfläche führt wegen der Volumenvergrößerung während der Phosgenierung zu einer Beschleunigung der Strömung. Durch eine in Strömungsrichtung geeignet gewählte Erweiterung der durchströmten Querschnittsfläche kann die Strömungsgeschwindigkeit der Reaktionsmischung über die Länge des Reaktors konstant gehalten werden. Dadurch erhöht sich die zur Verfügung stehende Reaktionszeit bei gleich bleibender Länge des Reaktors. Dieser Vorteil ist speziell bei der Umsetzung der relativ träge reagierenden aromatischen Amine von Bedeutung.

Für das erfindungsgemäße Verfahren können primäre Amine verwendet werden. Bevorzugt werden primäre Amine eingesetzt, die ohne Zersetzung in die Gasphase überführt werden können. Besonders geeignet sind Amine, insbesondere Diamine, auf Basis von aliphatischen oder cycloaliphatischen Kohlenwasserstoffen mit 1 bis 15 Kohlenstoffatomen. Besonders gut geeignete Amine sind 1,6-Diamino-hexan, 1-Amino-3,3,5-trimethyl-5-aminomethylcyclohexan (IPDA) und 4,4'-Diaminodicyclohexylamin. Bevorzugt verwendet wird 1,6-Diaminohexan (HDA).

Ebenfalls können für das erfindungsgemäße Verfahren aromatische Amine verwendet werden, die bevorzugt ohne Zersetzung in die Gasphase überführt werden können. Beispiele für bevorzugte aromatische Amine sind Toluylendiamin (TDA), insbesondere 2,4- TDA und 2,6-TDA und Gemische daraus, Diaminobenzol, Naphthyldiamin (NDA) und 2,2'-, 2,4'-oder 4,4'-Methylendiphenyldiamin (MDA) oder Isomerengemische davon. Besonders bevorzugt ist Toluylendiamin (TDA ), insbesondere 2,4-TDA und 2,6-TDA und Gemische daraus.

Die Ausgangsamine werden vor der Durchführung des erfindungsgemäßen Verfahrens in der Regel verdampft und auf 200°C bis 600°C , bevorzugt 201 bis 500, besonders bevorzugt 250°C bis 450°C erhitzt und gegebenenfalls verdünnt mit einem Inertgas wie N₂, He, Ar oder mit den Dämpfen eines inerten Lösungsmittels, z.B. aromatischen Kohlenwasserstoffen ggf. mit Halogensubstitution, wie z.B. Chlorbenzol oder o-Dichlorbenzol, dem Reaktionsraum zugeführt.

Die Verdampfung der Ausgangsamine kann in allen bekannten Verdampfungsapparaturen erfolgen, bevorzugt werden Verdampfungssysteme, bei denen ein kleiner Arbeitsinhalt mit einer hohen Umwälzleistung über einen Fallfilmverdampfer geführt, dabei zur Minimierung der thermischen Belastung der Ausgangsamine der Verdampfungsvorgang - wie oben ausgeführt- ggf. unter Zuspeisung von Inertgas und / oder Dämpfen eines inerten Lösungsmittels unterstützt wird. Die dampfförmigen Amine können noch Anteile an unverdampften Tröpfchen an Aminen (Aerosol) enthalten. Bevorzugt enthalten die dampfförmigen Amine jedoch im Wesentlichen keine Tröpfchen an unverdampften Aminen, das heißt maximal 0,5 Gew.-% des Amins, besonders bevorzugt maximal 0,05 Gew.-% des Amins, bezogen auf das Gesamtgewicht des Amins, liegt in der Form von unverdampften Tröpfchen vor und der restliche Teil des Amins liegt dampfförmig vor. Ganz besonders bevorzugt enthalten die dampfförmigen Amine keine Tröpfchen an unverdampften Aminen. Erfindungsgemäß wird nach der Verdampfung das dampfförmige Amin, ggf. mit Inertgasen oder inerten Lösungsmitteldämpfen verdünnt, über einen Nacherhitzer auf die gewünschte Einsatztemperatur gebracht.

Weiterhin bevorzugt erfolgt die Verdampfung und Überhitzung der Ausgangsamine mehrstufig, um unverdampfte Tröpfchen im dampfförmigen Aminstrom zu vermeiden. Insbesondere bevorzugt sind mehrstufige Verdampfungsschritte, in denen zwischen den Verdampfungs- und Überhitzungssystemen Tröpfchenabscheider eingebaut sind und / oder die Verdampfungsapparaturen auch die Funktion eines Tröpfchenabscheiders besitzen. Geeignete Tröpfchenabscheider sind z.B. beschrieben in "Droplet Separation", A. Bürkholz, VCH Verlagsgesellschaft, Weinheim - New York - Basel - Cambridge, 1989. Besonders bevorzugt sind Tröpfchenabscheider die einen geringen Druckverlust verursachen. Ganz besonders bevorzugt wird das verdampfte Amin über einen Nacherhitzer auf die gewünschte Einsatztemperatur gebracht, der auch als Tröpfchenabscheider fungiert. Insbesondere bevorzugt hat dieser Nacherhitzer einen Flüssigkeitsablauf um die stetige Entleerung des Abscheiders zu gewährleisten. Durch die Erzeugung eines im Wesentlichen Tröpfchen freien dampfförmigen Ausgangsaminstromes vor Eintritt in den Reaktor wird die Reaktorlaufzeit deutlich erhöht.

Beim erfindungsgemäßen Verfahren ist es vorteilhaft, Phosgen im Überschuss bezüglich Aminogruppen einzusetzen. Üblicherweise liegt ein molares Verhältnis von Phosgen zu Aminogruppen von 1,1 : 1 bis 20 : 1, bevorzugt 1,2 : 1 bis 5 : 1 vor. Auch das Phosgen wird auf Temperaturen von 200°C bis 600°C erhitzt und gegebenenfalls verdünnt mit einem Inertgas wie N₂, He, Ar oder mit den Dämpfen eines inerten Lösungsmittels, z.B. aromatischen Kohlenwasserstoffen ohne oder mit Halogensubstitution, wie z.B. Chlorbenzol oder o-Dichlorbenzol, dem Reaktionsraum zugeführt.

Das erfindungsgemäße Verfahren wird so durchgeführt, dass die getrennt aufgeheizte Reaktionspartner in wenigstens einen Reaktionsraum eingeführt, vermischt und unter Beachtung geeigneter Reaktionszeiten unter adiabater Reaktionsführung umgesetzt werden. Anschließend wird durch Abkühlung des Gasstromes das Isocyanat kondensiert, wobei die Abkühlung bis zu einer Temperatur oberhalb der Zersetzungstemperatur des entsprechenden Carbamidsäurechlorids, also beispielsweise Toluylendiaminsäurechlorid im Falle von TDA, erfolgt.

Die notwendige Verweilzeit zur Reaktion der Amingruppen mit dem Phosgen zu Isocyanat liegt zwischen 0,05 und 15 Sekunden, in Abhängigkeit von der Art des eingesetzten Amins, der Starttemperatur, der adiabaten Temperaturerhöhung im Reaktionsraum, dem molaren Verhältnis von eingesetztem Amin und Phosgen und einer etwaigen Verdünnung der Reaktionspartner mit Inertgasen.

Wird für das jeweilige System ( Starttemperatur, adiabate Temperaturerhöhung, molares Verhältnis der Reaktanden, Verdünnungsgas, eingesetztes Amin ) eine einmal ermittelte Mindestverweilzeit für die vollständige Reaktion um weniger als 20%, vorzugsweise weniger als 10% überschritten, kann die Bildung von Folge-Reaktionsprodukten wie Isocyanuraten und Carbodiimiden weitgehend vermieden werden.

Innerhalb dieses für chemische Reaktionen sehr engen Kontaktzeitspektrums muss sowohl die möglichst homogene Vermischung der Reaktionspartner als auch die weitere Reaktion erfolgen. Dabei erfolgt die weitere Reaktion bevorzugt ohne Rückvermischung, die eine Verbreiterung des Kontaktzeitraums und damit eine vermehrte Bildung von unerwünschten Neben- und Folgeprodukten bewirken würde.

Bei der praktischen Durchführung des Verfahrens kann eine Abweichung von der mittleren Kontaktzeit durch die notwendige Zeit der Vermischung der Reaktionspartner erfolgen. Solange die Reaktionspartner noch nicht homogen vermischt sind, sind im Reaktor noch unvermischte oder teilvermischte Gasvolumina vorhanden, in denen noch kein oder noch kein vollständiger Kontakt der Reaktionspartner erfolgt ist. Die Vermischung der Reaktionspartner soll daher bevorzugt innerhalb einer Zeit von 0,01 bis 0,3 Sekunden bis zu einem Segregationsgrad von mindestens 10⁻¹ erfolgen. Der Segregationsgrad ist ein Maß für die Unvollständigkeit der Vermischung (siehe z.B. Chem.-Ing.-Techn. 44, (1972), S. 1051ff; Appl.SCi.Res.(The Hague) A3 (1953), S.279).

Die Methoden zur Durchführung kurzer Mischzeiten sind im Prinzip bekannt. Geeignet sind beispielsweise Mischaggregate oder Mischzonen mit bewegten oder statischen Mischorganen oder Düsen. Bevorzugt sind statische Mischer, wie sie z.B. in EP-A-1 362 847, EP-A-1 526 129 oder EP-A- 1 555 258 beschrieben sind.

Nach Vermischung der Reaktionskomponenten durchströmt das Reaktionsgemisch den Reaktionsraum. Dabei weist weder die Mischzone noch der daran anschließende Reaktionsraum Heizflächen auf, die Anlass zu einer thermischen Belastung mit der Folge von Folgereaktionen wie Isocyanurat-oder Carbodiimidbildung geben können, oder Kühlflächen auf, die Anlass einer Kondensation mit der Folge von Ablagerungen geben können. Die Komponenten werden adiabat umgesetzt, dabei wird die adiabate Temperaturerhöhung im Reaktor allein über die Temperaturen, Zusammensetzungen und relativen Dosierungen der Eduktströme sowie die Verweilzeit im Reaktor eingestellt.

Die Durchströmung des Reaktionsraums soll bevorzugt in Form einer zu 90% angenäherten Pfropfenströmung erfolgen, so dass alle Volumenteile der Strömung angenähert die gleiche Strömungszeit aufweisen, damit eine möglichst geringe weitere Verbreiterung der Kontaktzeitverteilung zwischen den Reaktionspartnern erfolgt. Der Grad der Realisierung der idealen Pfropfenströmung (mit einer mittleren Abweichung von der mittleren Verweilzeit = 0) wird in der Strömungstechnik durch die Bodenstein-Zahl Bo (Fitzer, Techn. Chemie, Springer 1989, S. 288-295) beschrieben. Bevorzugt soll die Bodenstein-Zahl im erfindungsgemäßen Verfahren mindestens 10, bevorzugt größer 100, besonders bevorzugt größer 250 betragen.

Nach der erfolgten Phosgenierungsreaktion im Reaktionsraum wird in Schritt c) das den Reaktionsraum kontinuierlich verlassende, gasförmige Gemisch, das bevorzugt wenigstens ein Isocyanat, Phosgen und Chlorwasserstoff umfasst, von dem gebildeten Isocyanat befreit. Dies kann beispielsweise einstufig durch selektive Kondensation in einem inerten Lösungsmittel erfolgen, wie es bereits für andere Gasphasenphosgenierungen empfohlen wurde (EP-A-0 749 958).

Bevorzugt erfolgt die Kondensation jedoch dadurch, dass in das den Reaktionsraum verlassende Gasgemisch ein oder mehrere geeignete Flüssigkeitsströme (Quench-Flüssigkeiten) eingesprüht werden. Dadurch kann, wie in EP-A- 1 403 248 beschrieben, eine rasche Abkühlung des Gasgemisches ohne den Einsatz kalter Flächen durchgeführt werden. Unabhängig von der Art der gewählten Abkühlung wird die Temperatur der Abkühlzone jedoch bevorzugt so gewählt, dass sie einerseits oberhalb der Zersetzungstemperatur des dem Isocyanat entsprechenden Carbamidsäurechlorids liegt und andererseits das Isocyanat und gegebenenfalls das in dem Amindampfstrom und / oder Phosgenstrom als Verdünnungsmittel mit verwendete Lösungsmittel kondensieren bzw. sich in dem Lösungsmittel lösen, während überschüssiges Phosgen, Chlorwasserstoff und ggf. als Verdünnungsmittel mit verwendetes Inertgas die Kondensations- bzw. Quenchstufe durchlaufen. Zur selektiven Gewinnung des Isocyanats aus dem den Reaktionsraum gasförmig verlassenden Gemisch besonders gut geeignet sind bei einer Temperatur von 80 bis 200°C, vorzugsweise 80 bis 180 °C gehaltene Lösungsmittel wie beispielsweise Chlorbenzol und / oder Dichlorbenzol, oder in diesem Temperaturbereich gehaltenes Isocyanat oder Mischungen des Isocyanats mit Chlorbenzol und / oder Dichlorbenzol.

Die Erzeugung der für das erfindungsgemäße Verfahren wesentlichen Strömung des gasförmigen Reaktionsgemischs als weitgehende Pfropfenströmung ohne wesentliche Rückvermischung ausgehend von der Mischzone durch den Reaktionsraum wird durch ein Druckgefälle zwischen den E-duktzuleitungen zur Mischzone einerseits und dem Ausgang der Kondensations- bzw. Quenchstufe andererseits sichergestellt. Im Allgemeinen liegt der absolute Druck in den Zuleitungen zu der Mischzone bei 200 bis 3000 mbar und hinter der Kondensations- bzw. Quenchstufe bei 150 bis 2500 mbar. Wesentlich ist hierbei jedoch lediglich die Aufrechterhaltung eines Differenzdrucks zwecks Gewährleistung der genannten gerichteten Strömung.

Das die Kondensations- bzw. Quenchstufe verlassende Gasgemisch wird in Schritt d) in einer nachgeschalteten Gaswäsche mit einer geeigneten Waschflüssigkeit von restlichem Isocyanat befreit, anschließend in an sich bekannter Weise von überschüssigem Phosgen befreit. Dies kann mittels einer Kühlfalle, Absorption in einem inerten Lösungsmittel (z.B. Chlorbenzol oder Dichlorbenzol) oder durch Adsorption und Hydrolyse an Aktivkohle erfolgen. Das die Phosgenrückgewinnungsstufe durchlaufende Chlorwasserstoffgas kann in an sich bekannter Weise zur Rückgewinnung des zur Phosgensynthese erforderlichen Chlors recyclisiert werden. Die in Schritt d) nach ihrem Einsatz zur Gaswäsche anfallende Waschflüssigkeit wird dann bevorzugt als Quench-Flüssigkeit zur Abkühlung des aus dem Rohrreaktor austretenden Gasgemisches in Schritt c) eingesetzt.

Die Reindarstellung der Isocyanate erfolgt bevorzugt anschließend durch destillative Aufarbeitung der Lösungen bzw. Gemische aus der Kondensations- bzw. Quenchstufe.

### Beispiele:

### Beispiel 1"nicht-adiabate Phosgenierung von TDA" (nicht erfindungsgemäß):

20 kg/h eines Gemisches bestehend aus 2,4- und 2,6-Toluylendiamin im Gewichts-Verhältnis von 80% zu 20% werden verdampft und gasförmig mit 400°C einem Rohrreaktor zugeleitet. Gleichzeitig werden parallel dazu 100 kg/h gasförmiges Phosgen auf 310°C erwärmt und ebenfalls dem Rohrreaktor zugeführt. Die Ströme werden durch eine Düse in die Mischzone eingedüst und gemischt und treten in den Reaktionsraum ein. Die Mischzone ist isoliert, um Wärmeverluste vor und während der Vermischung zu verhindern. Der Reaktionsraum ist nicht wärmeisoliert und wird durch Wärmeabstrahlung gekühlt. Die Reaktionsführung ist somit nicht-adiabat. Das den Rohrreaktor nach 2,2 Sekunden verlassende Gasgemisch weist eine Endtemperatur von 380 °C auf und wird durch Eindüsen von ortho-Dichlorbenzol gekühlt und das gebildete Isocyanat kondensiert und ausgewaschen sowie anschließend nach bekannten Methoden destillativ aufgearbeitet. Die Druckdifferenz zwischen dem Druck in der TDA-Zuleitung und der Kondensationsstufe beträgt 200 mbar, um eine gerichtete Gasströmung von den Zuleitungen zur Mischzone bis zur Kondensationsstufe zu erreichen. Nach 96 h Reaktionszeit steigt der Druck in der TDA-Zuleitung stark an, da der Reaktionsraum im Rohrreaktor durch bei der Reaktion gebildete Ablagerungen an den Rohrwänden verengt ist. Die Bildung der Ablagerungen ist auf verstärkte Nebenproduktbildung zurückzuführen. Der Versuch muss daher abgebrochen werden.

### Beispiel 2 "adiabate Phosgenierung von TDA" (erfindungsgemäß):

20.5 kmol/h eines Gemisches bestehend aus 2,4 und 2,6-Toluylendiamin im Gewichts-Verhältnis von 80% zu 20% werden zusammen mit 500 kg/h Stickstoff verdampft und gasförmig einem Rohrreaktor mit einer Temperatur von 320 °C zugeleitet. Gleichzeitig werden parallel dazu 182 kmol/h gasförmiges Phosgen zusammen mit 1000 kg/h ortho-Dichlorbenzol auf 360°C erwärmt und ebenfalls dem Rohrreaktor zugeführt. Die Ströme werden durch eine Düse in die Mischzone eingedüst und vermischt und treten in den Reaktionsraum ein. Die Mischzone und der Reaktionsraum sind wärmeisoliert, so dass weder ein zusätzlicher Wärmeeintrag durch Heizung noch eine Wärmeabfuhr durch externe Kühlung oder Wärmeabstrahlung erfolgt. Die Reaktion wird somit unter adiabatischen Bedingungen durchgeführt. Es wird eine Endtemperatur von 405°C durch Auflagethermometer am Austritt des Reaktionsraumes gemessen. Das den Reaktionsraum nach 5,5 Sekunden verlassende Gasgemisch wird durch Eindüsen von ortho-Dichlorbenzol gekühlt und das gebildete Isocyanat kondensiert und ausgewaschen sowie anschließend nach bekannten Methoden destillativ aufgearbeitet. Die Druckdifferenz zwischen dem Druck in der TDA-Zuleitung und der Kondensationsstufe beträgt 60 mbar, um eine gerichtete Gasströmung von den Zuleitungen zur Mischzone bis zur Kondensationsstufe zu erreichen. Auch nach 720 h Reaktionszeit wird kein Anstieg der Druckdifferenz gemessen, was auf eine Reaktion ohne Bildung von Ablagerungen hinweist. Eine Inspektion des Reaktionraumes zeigt ebenfalls keine Hinweise auf eine Rückstandsbildung.

### Beispiel 3 ,,adiabate Phosgenierung von IPDA" (erfindungsgemäß)

17,6 kmol/h Isophorondiamin werden zusammen mit 42 kg/h Stickstoff verdampft und überhitzt auf eine Temperatur von 300°C gasförmig einem Rohrreaktor zugeleitet. Gleichzeitig wird parallel dazu 64 kmol/h gasförmiges Phosgen auf 300°C erwärmt und ebenfalls dem Rohrreaktor zugeführt. Die Ströme werden innerhalb einer Mischzeit von 0,02sec vermischt und treten in den Reaktionsraum ein. Die Mischzone und der Reaktionsraum sind wärmeisoliert, so dass weder ein zusätzlicher Wärmeeintrag durch Heizung noch eine Wärmeabfuhr durch externe Kühlung oder Wärmeabstrahlung erfolgt. Die Reaktion wird somit unter adiabatischen Bedingungen durchgeführt. Es wird eine Endtemperatur von 450°C durch Auflagethermometer am Austritt des Reaktionsraumes gemessen. Das den Reaktionsraum nach 0,1 s verlassende Gasgemisch wird durch Eindüsen von Monochlorbenzol gekühlt und das gebildete Isocyanat kondensiert und ausgewaschen und anschließend nach bekannten Methoden destillativ aufgearbeitet. Die Druckdifferenz zwischen dem Druck in der IPDA-Zuleitung und der Kondensationsstufe beträgt 200 mbar, und die Druckdifferenz zwischen der Phosgen-Zuleitung und der Kondensationsstufe 40 mbar, um eine gerichtete Gasströmung von den Zuleitungen zur Mischzone bis zur Kondensationsstufe zu erreichen. Auch nach 1000 h Reaktionszeit wird kein Druckanstieg beobachtet. Bei der anschließenden Inspektion des Reaktionraumes werden keine nennenswerten Rückstandsbeläge gefunden.

## Patentansprüche

1. Verfahren zur Herstellung von Isocyanaten durch Umsetzung entsprechender primärer Amine mit Phosgen, worin die primären Amine verdampft und nach der Verdampfung die dampfförmigen Amine über einen Nacherhitzer auf die Einsatztemperatur gebracht werden, **dadurch gekennzeichnet, dass** Phosgen und die primären Amine oberhalb der Siedetemperatur der Amine innerhalb einer mittleren Kontaktzeit von 0,05 bis 15 Sekunden umgesetzt werden, wobei die Umsetzung adiabat durchgeführt wird.

2. Verfahren nach Anspruch 1, bei dem anschließend das Isocyanat durch Abkühlung des Gasstromes kondensiert wird, wobei die Abkühlung bis zu einer Temperatur oberhalb der Zersetzungstemperatur des entsprechenden Carbamidsäurechlorids erfolgt, und anschließend das Gasgemisch von überschüssigem Phosgen befreit wird, und anschließend das Chlorwasserstoffgas zur Rückgewinnung des zur Phosgensynthese erforderlichen Chlors recyclisiert wird.

3. Verfahren nach einem der Ansprüche 1 oder 2, bei dem die Amine vor der Reaktion verdampft und, gegebenenfalls verdünnt mit einem Inertgas oder mit den Dämpfen eines inerten Lösungsmittels, auf Temperaturen von 200 bis 600 °C erhitzt werden, wobei die verdampften Amine maximal 0,5 Gew.-% Tröpfchen an unverdampftem Amin, bezogen auf das Gesamtgewicht des Amins, enthalten.

4. Verfahren nach einem der Ansprüche 1 bis 3, **dadurch gekennzeichnet, dass** man
a) die dampfförmigen Amine, gegebenenfalls verdünnt mit einem Inertgas oder mit den Dämpfen eines inerten Lösungsmittels, und Phosgen getrennt auf Temperaturen von 200 bis 600 °C erhitzt und kontinuierlich vermischt,
b) das in Schritt a) erzeugte Reaktionsgemisch unter Vermeidung von Rückvermischung kontinuierlich durch einen Reaktionsraum leitet und es darin innerhalb einer mittleren Kontaktzeit von 0,05 bis 15 Sekunden adiabat umsetzt,
c) das aus dem Reaktionsraum austretende Gasgemisch zur Kondensation des gebildeten Isocyanats abkühlt, wobei die Temperatur oberhalb der Zersetzungstemperatur der den umgesetzten Aminen entsprechenden Carbamidsäurechloride gehalten wird, und
d) nicht kondensiertes Isocyanat aus dem Gasgemisch durch Waschen mit einer Waschflüssigkeit abtrennt.

5. Verfahren nach Anspruch 4, **dadurch gekennzeichnet, dass** man in Schritt b) das in Schritt a) erzeugte Reaktionsgemisch durch einen Reaktionsraum leitet, der eine rotationssymmetrische Geometrie mit einer in Strömungsrichtung des Reaktionsgemischs konstanten oder steigenden durchströmten Querschnittsfläche aufweist.

6. Verfahren nach Anspruch 4 oder 5, **dadurch gekennzeichnet, dass** man in Schritt b) das in Schritt a) erzeugte Reaktionsgemisch durch einen Reaktionsraum leitet, der in Strömungsrichtung Abschnitte konstanter und steigender Querschnittsfläche aufweist.

7. Verfahren nach einem der Ansprüche 4 bis 6, **dadurch gekennzeichnet, dass** das aus dem Reaktionsraum austretende Gasgemisch wenigstens ein Isocyanat, Phosgen und Chlorwasserstoff umfasst und in Schritt c) durch Einsprühen wenigstens eines Flüssigkeitsstroms in das Gasgemisch abgekühlt wird.

8. Verfahren nach Anspruch 7, **dadurch gekennzeichnet, dass** zur Abkühlung des aus dem Reaktionsraum austretenden Gasgemisches in Schritt c) zumindest teilweise die in Schritt d) nach ihrem Einsatz zur Gaswäsche anfallende Waschflüssigkeit eingesetzt wird.

9. Verfahren nach Anspruch 7, **dadurch gekennzeichnet, dass** zur Abkühlung des aus dem Reaktionsraum austretenden Gasgemisches in Schritt c) zumindest teilweise das in Schritt c) nach Kondensation erhaltene Gemisch eingesetzt wird.

10. Verfahren nach einem der Ansprüche 1 bis 9, **dadurch gekennzeichnet, dass** die Isocyanate Toluylendiisocyanat, Methylendiphenyldiisocyanat, Dicyclohexylmethandiisocyanat, Hexamethylendiisocyanat und / oder Isophorondiisocyanat sind.

11. Verfahren nach Anspruch 3, **dadurch gekennzeichnet, dass** die verdampften Amine keine Tröpfchen an unverdampftem Amin enthalten.

## Claims

1. Process for preparing isocyanates by reaction of corresponding primary amines with phosgene, in which the primary amines are vaporized and after vaporization the gaseous amines are brought to the input temperature by means of an after-heater, **characterized in that** phosgene and the primary amines are reacted at above the boiling point of the amines within an average contact time of from 0.05 to 15 seconds, with the reaction being carried out adiabatically.

2. Process according to Claim 1, wherein the isocyanate is subsequently condensed by cooling the gas stream, with cooling being carried out down to a temperature above the decomposition temperature of the corresponding carbamoyl chloride and the gas mixture is subsequently freed of excess phosgene and the hydrogen chloride gas is subsequently recirculated for recovery of the chlorine necessary for the synthesis of phosgene.

3. Process according to either of Claims 1 and 2, wherein the amines are vaporized before the reaction and, optionally diluted with an inert gas or with the vapour of an inert solvent, heated to temperatures of from 200 to 600°C, where the vaporized amines contain a maximum of 0.5% by weight of droplets of unvaporized amine, based on the total weight of the amine.

4. Process according to any of Claims 1 to 3, **characterized in that**
a) the gaseous amines, optionally diluted with an inert gas or with the vapour of an inert solvent, and phosgene are separately heated to temperatures of from 200 to 600°C and continuously mixed,
b) the reaction mixture produced in step a) is passed continuously with avoidance of backmixing through a reaction space and reacted adiabatically therein within an average contact time of from 0.05 to 15 seconds,
c) the gas mixture leaving the reaction space is cooled to condense the isocyanate formed, with the temperature being kept above the decomposition temperature of the carbamoyl chlorides corresponding to the amines reacted, and
d) uncondensed isocyanate is separated off from the gas mixture by washing with a washing liquid.

5. Process according to Claim 4, **characterized in that** the reaction mixture produced in step a) is, in step b), passed through a reaction space which has a rotationally symmetric geometry having a cross-sectional area through which flow occurs which is constant or increases in the flow direction of the reaction mixture.

6. Process according to Claim 4 or 5, **characterized in that** the reaction mixture produced in step a) is, in step b), passed through a reaction space which has sections having a cross-sectional area which is constant or increases in the flow direction.

7. Process according to any of Claims 4 to 6, **characterized in that** the gas mixture leaving the reaction space comprises at least an isocyanate, phosgene and hydrogen chloride and is cooled in step c) by spraying at least one liquid stream into the gas mixture.

8. Process according to Claim 7, **characterized in that** the cooling in step c) of the gas mixture leaving the reaction space is carried out at least partly using the washing liquid obtained in step d) after its use for washing the gas.

9. Process according to Claim 7, **characterized in that** the cooling in step c) of the gas mixture leaving the reaction space is carried out at least partly using the mixture obtained in step c) after condensation.

10. Process according to any of Claims 1 to 9, **characterized in that** the isocyanates are tolylene diisocyanate, methylenedi(phenyl isocyanate), dicyclohexylmethane diisocyanate, hexamethylene diisocyanate and/or isophorone diisocyanate.

11. Process according to Claim 3, **characterized in that** the vaporized amines do not contain any droplets of unvaporized amine.

## Revendications

1. Procédé pour la préparation d'isocyanates par transformation d'amines primaires correspondantes avec du phosgène, dans lequel les amines primaires sont évaporées et après l'évaporation, les amines sous forme de vapeur sont amenées à la température d'utilisation via un post-réchauffeur, **caractérisé en ce que** le phosgène et les amines primaires sont transformés au-dessus de la température d'ébullition des amines en un temps de contact moyen de 0,05 à 15 secondes, la transformation étant réalisée de manière adiabatique.

2. Procédé selon la revendication 1, dans lequel l'isocyanate est ensuite condensé par refroidissement du flux gazeux, le refroidissement ayant lieu jusqu'à une température supérieure à la température de décomposition du chlorure de l'acide carbamique correspondant, puis le mélange gazeux est libéré du phosgène en excès et ensuite, le chlorure d'hydrogène gazeux est recyclé en vue de la récupération du chlore nécessaire à la synthèse du phosgène.

3. Procédé selon l'une quelconque des revendications 1 ou 2, dans lequel les amines sont évaporées avant la réaction et chauffées, le cas échéant sous forme diluée par un gaz inerte ou par les vapeurs d'un solvant inerte, à des températures de 200 à 600°C, les amines évaporées contenant au maximum 0,5% en poids de gouttes d'amine non évaporée, par rapport au poids total de l'amine.

4. Procédé selon l'une quelconque des revendications 1 à 3, **caractérisé en ce qu'**on
a) chauffe les amines sous forme vapeur, le cas échéant sous forme diluée par un gaz inerte ou par les vapeurs d'un solvant inerte, et le phosgène séparément à des températures de 200 à 600°C et les mélange en continu,
b) fait passer le mélange réactionnel produit dans l'étape a), en évitant un mélange en retour, en continu dans une chambre de réaction et on le transforme dans celle-ci en un temps de contact moyen de 0,05 à 15 secondes de manière adiabatique,
c) refroidit le mélange gazeux sortant de la chambre de réaction, en vue de la condensation de l'isocyanate formé, la température étant maintenue au-dessus de la température de décomposition des chlorures d'acide carbamique correspondant aux amines transformées, et
d) sépare l'isocyanate non condensé du mélange gazeux par lavage avec un liquide de lavage.

5. Procédé selon la revendication 4, **caractérisé en ce qu'**on fait passer dans l'étape b) le mélange réactionnel produit dans l'étape a) dans une chambre de réaction qui présente une géométrie symétrique en rotation présentant une section transversale traversée constante ou croissante dans le sens d'écoulement du mélange réactionnel.

6. Procédé selon la revendication 4 ou 5, **caractérisé en ce qu'**on fait passer dans l'étape b) le mélange réactionnel produit dans l'étape a) dans une chambre de réaction qui présente des portions de section transversale constante et croissante dans le sens d'écoulement.

7. Procédé selon l'une quelconque des revendications 4 à 6, **caractérisé en ce que** le mélange gazeux sortant de la chambre de réaction comprend au moins un isocyanate, du phosgène et du chlorure d'hydrogène et est refroidi dans l'étape c) par injection d'au moins un flux de liquide dans le mélange gazeux.

8. Procédé selon la revendication 7, **caractérisé en ce que** pour le refroidissement du mélange gazeux sortant de l'espace de réaction dans l'étape c), on utilise au moins partiellement le liquide de lavage obtenu dans l'étape d) après son utilisation pour le lavage du gaz.

9. Procédé selon la revendication 7, **caractérisé en ce que** pour le refroidissement du mélange gazeux sortant de l'espace de réaction dans l'étape c), on utilise au moins partiellement le mélange obtenu dans l'étape c) après condensation.

10. Procédé selon l'une quelconque des revendications 1 à 9, **caractérisé en ce que** les isocyanates sont le toluylènediisocyanate, le méthylènediphényldiisocyanate, le dicyclohexylméthanediisocyanate, l'hexaméthylènediisocyanate et/ou l'isophoronediisocyanate.

11. Procédé selon la revendication 3, **caractérisé en ce que** les amines évaporées ne contiennent pas de gouttes d'amine non évaporée.
